(19) Europäisches Patentamt
European Patent Office
Office européen des brevets

(11) Veröffentlichungsnummer: **0 501 250 A1**

(12) **EUROPÄISCHE PATENTANMELDUNG**

(21) Anmeldenummer: **92102449.3**

(22) Anmeldetag: **14.02.92**

(51) Int. Cl.5: **C07K 9/00**, C07D 487/04, A61K 37/02

(30) Priorität: **27.02.91 DE 4106101**

(43) Veröffentlichungstag der Anmeldung: **02.09.92 Patentblatt 92/36**

(84) Benannte Vertragsstaaten: **AT BE CH DE DK ES FR GB GR IT LI LU NL SE**

(71) Anmelder: **BAYER AG**

**W-5090 Leverkusen 1 Bayerwerk(DE)**

(72) Erfinder: **Lerchen, Hans-Georg, Dr.**
**Morgengraben 14**
**W-5000 Köln 80(DE)**
Erfinder: **Baumgarten, Jörg, Dr.**
**Henselweg 13**
**W-5600 Wuppertal 1(DE)**
Erfinder: **Piel, Norbert, Dr.**
**Neuenhausstrasse 28**
**W-4006 Erkrath 1(DE)**
Erfinder: **Lorentzen, Jens-Peter, Dr.**
**An der Ruthen 2**
**W-5000 Köln 80(DE)**
Erfinder: **Antonicek, Horst-Peter, Dr.**
**Wiesenwinkel 14**
**W-5060 Bergisch Gladbach(DE)**
Erfinder: **Kabbe, Hans-Joachim, Dr.**
**Walter-Flex-Strasse 16**
**W-5090 Leverkusen 1(DE)**

(54) **Fucose-markierte Batracylinderivate.**

(57) Das heterocyclische Amin Batracylin zeigt ausgezeichnete Antitumorwirkung im Colon-38-Modell an der Maus; dieses Modell gilt als repräsentativ für die Wirkung gegen Dickdarmkrebs. Diese Wirkung läßt sich durch eine Markierung mit Fucose verbessern.

Die neuen Verbindungen haben die Formel:

$[R]n-AA_1-AA_2-C$,

wo:

C = Batracylin

$AA_1$ und $AA_2$ = gegebenenfalls geschützte L- oder D-Aminosäuren

n = 0-2

;

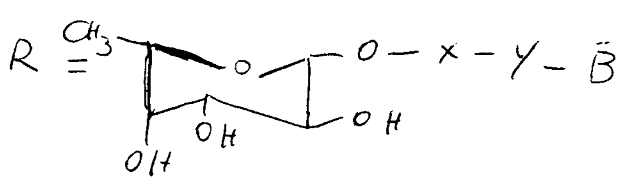

x = Alkylen oder Arylen

y = CO, NH, O

B = Bindung oder Diacylrest einer Dicarbonsäure.

EP 0 501 250 A1

Das heterocyclische Amin 1 (Batracylin) zeigt ausgezeichnete Antitumorwirkung im Colon-38-Modell an der Maus; dieses Modell gilt als repräsentativ für die Wirkung gegen Dickdarmkrebs. (US 4 757 072).

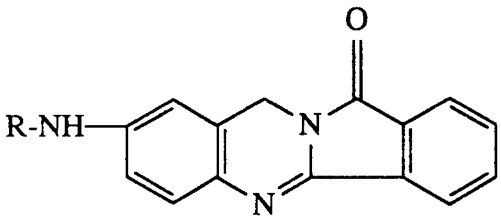

1 R = H US 4 757 072

2 R = 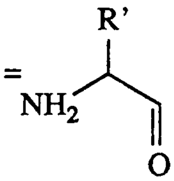 US 4 180 343

Die Wirkung von Batracylin ist stark eingeschränkt durch die geringe Löslichkeit in wasserhaltigen Lösungsmitteln, die u.a. eine Herstellung applizierbarer Lösungen erschwert.

Dieses Handikap wurde durch die Acylierung von 1 mit natürlichen Aminosäuren zu den Acylderivaten 2 überwunden (US 4 180 343). Verbindungen des Typs 2 werden durch Peptidasen in Humanserum zu 1 gespalten und sind daher, bei besserer Wasserlöslichkeit, ebenso wirksam wie 1.

Im Rahmen von weiteren Untersuchungen wurde nun überraschend gefunden, daß neue Peptid- und Glycopeptidkonjugate von 1, die keine Prodrugs darstellen, an SW 480-Zellinien cytostatische Wirkung zeigen, die der von Batracylin 1 gleich kommt oder diese sogar übertrifft.

Interessanterweise führte die Anknüpfung von β-L-Fucose-Derivaten an Peptidkonjugate von 1 zu wirksamen Verbindungen, während alle anderen getesteten Glycopeptidderivate unwirksam waren.

Darüber hinaus konnte gezeigt werden, daß durch Anknüpfung von β-L-Fucose an nicht wirksame Peptidkonjugate die gewünschte Wirkung wiederhergestellt werden konnte.

Die neuen Derivate zeichnen sich durch günstigere Lösungseigenschaften aus und für β-L-Fucose-Konjugate können möglicherweise bessere Selektivitäten bezüglich der Wirkung erwartet werden.

Die neuen Verbindungen werden durch folgende allgemeine Formel beschrieben:

$[R]_n—AA_1—AA_2—C$

mit

C = ein Cytostatikum, bevorzugt

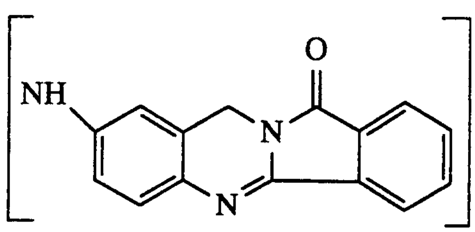,

R =

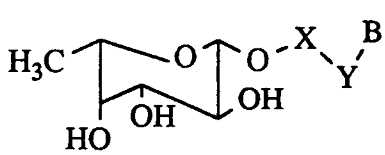,

X = Alkylen, Arylen

Y = CO,NH,O

B = Bindung oder Diacylrest einer Dicarbonsäure,

$AA_1$ = ist ein natürlicher Aminosäure-Rest in der L- oder D-Konfiguration oder Bindung. Falls $AA_1$ noch weitere funktionelle Gruppen trägt, so können diese deblockiert oder mit bekannten Schutzgruppen geschützt oder amidartig mit B verknüpft vorliegen. Als Schutzgruppen geeignet sind z.B. Benzyloxycarbonyl, Fluorenylmethoxycarbonyl, t-Butyloxycarbonyl, Benzyl oder tert.-Butyl.

$AA_2$ = ist eine natürliche Aminosäure in der D- oder L-Konfiguration oder Bindung. Falls $AA_2$ noch weitere funktionelle Gruppen trägt, so können diese deblockiert oder mit bekannten Schutzgruppen geschützt oder amidartig mit B verknüpft vorliegen. Als Schutzgruppen geeignet sind z.B. Benzyloxycarbonyl, Fluorenylmethoxycarbonyl, t-Butyloxycarbonyl, Benzyl oder tert.-Butyl.

n = 0,1,2.

Bevorzugt sind Verbindungen, bei denen

X = p-Phenylen oder$(CH_2)_m$,
wobei m 1-20, bevorzugt 1-10, sein kann

Y = CO,NH,

B = Bindung oder der Rest einer $C_3$-$C_6$-Dicarbonsäure,

n = 0,1,2.

Ganz besonders bevorzugt sind

für $AA_1$ Aminosäure-Reste mit sauren oder basischen Seitenketten, oder Bindung

für $AA_2$ Aminosäure-Reste wie Ala, Val, Leu, Ile, Nva, Gly, Phe (D- oder L-Konfiguration), die amidartig mit dem Heterocyclus und mit $AA_1$ verknüpft sind.

für R die folgenden $\beta$-L-Fucose-Derivate:

$R_1$ =

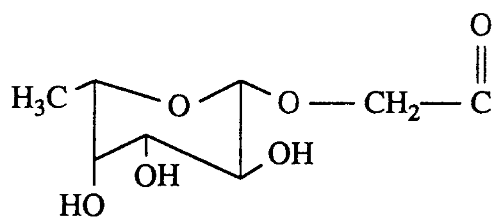

ß-L-Fuc-Hyac-

$R_2$ =

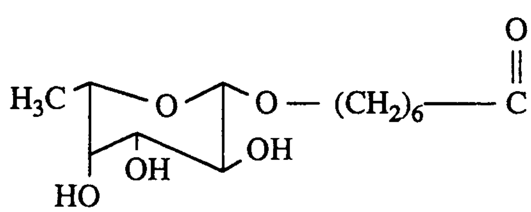

ß-L-Fuc-Hycapr-

$R_3$ =

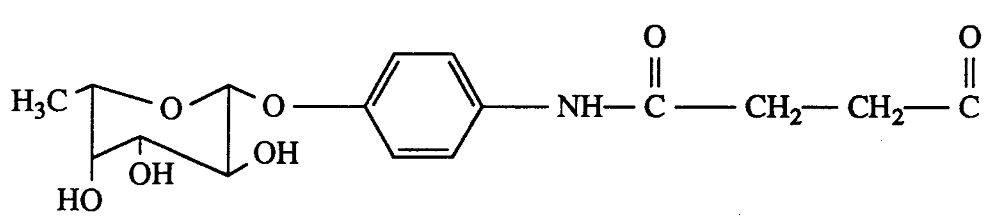

ß-L-Fuc-pAP-Suc-

$R_4$ =

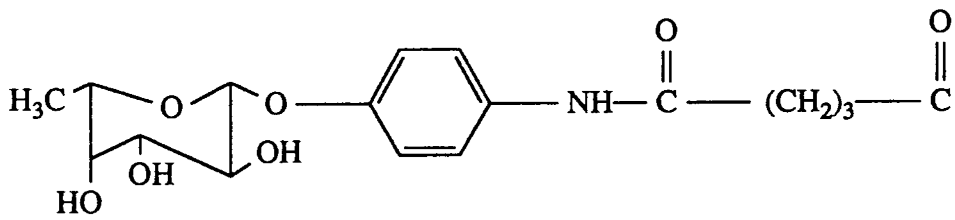

ß-L-Fuc-pAP-Glut-

$R_5$ =

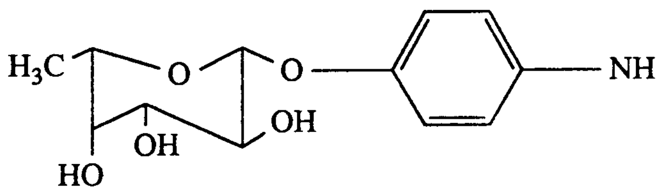

ß-L-Fuc-pAP-

$R_6$ =

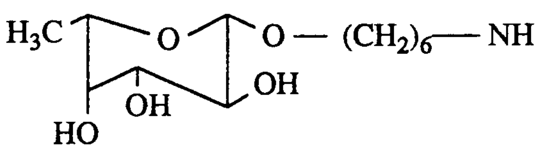

ß-L-Fuc-ω-AHex-

$R_7$ =

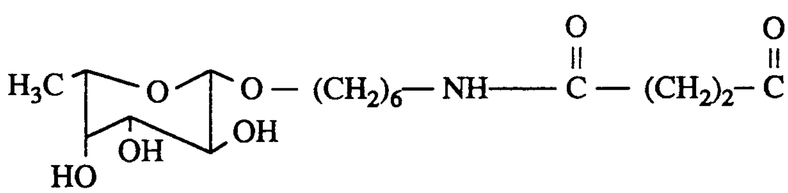

ß-L-Fuc-ω-AHex-Suc-

Anstelle des Batracylins können auch andere Cytostatika oder deren Derivate eingesetzt werden wie z.B.

Methotrexat:

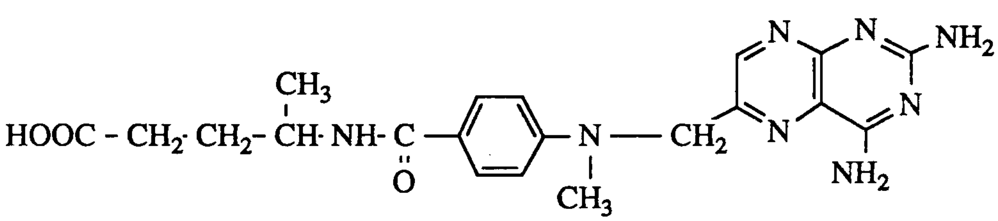

Nimestin:

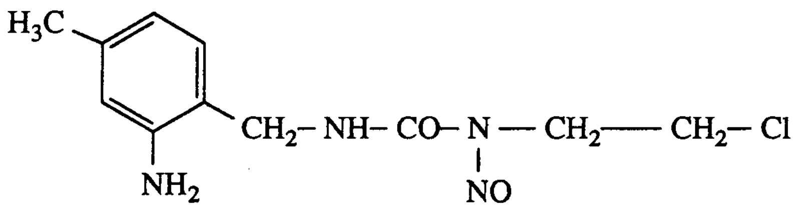

Daunorubicin:

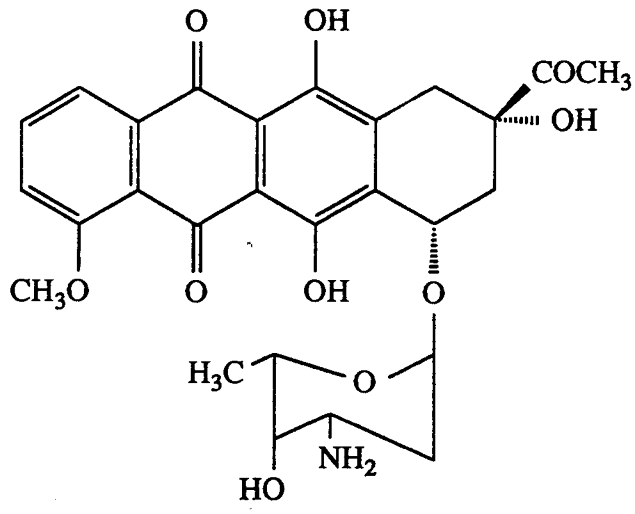

Abkürzungen und Allgemeines

Alle Aminosäuren werden in der IUPAC-IUB-Abkürzung (3 Buchstaben-Code) dargestellt.
Wenn keine Konfiguration angegeben ist, liegen sie in der L-Form vor.
Schutzgruppen werden in allgemein gebräuchlicher Schreibweise abgekürzt.

Weitere Abkürzungen:

| | |
|---|---|
| β-L-Fuc | β-L-Fucosyl |
| Hyac | Hydroxyacetat |
| Hycapr | ω-Hydroxycaprylat |
| pAP | p-Aminophenyl |
| Suc | Succinyl |
| Bzl | Benzyl |
| Z | Benzyloxycarbonyl |
| Fmoc | Fluorenylmethoxycarbonyl |
| Boc | t-Butyloxycarbonyl |
| DMF | N,N-Dimethylformamid |
| Ac | Acetyl |
| Hex | Hexyl |
| Glut | Glutarat |
| ω-AHex | ω-Amino-hexanyl(1) |
| DCC | Dicyclohexylcarbodiimid |
| HOSu | N-Hydroxysuccinimid |

Allgemeine Synthesevorschrift für Batracylin-Glycopeptidkonjugate

$\beta$-L-Fucoside mit $\omega$-Hydroxycarbonsäureestern oder geeignet an der Aminofunktion geschützten $\omega$-Hydroxyalkylaminen werden vorteilhaft aus peracylierter L-Fucose unter der Verwendung von Lewis-Säuren wie $(CH_3)_3SiOSO_2CF_3$, $BF_3 \cdot OEt_2$, $ZnCl_2$etc. oder aus acylierten L-Fucosylhalogeniden unter Verwendung von Schwermetallsalzen, bevorzugt Silber- oder Quecksilberverbindungen, in einem geeigneten Lösungsmittel wie z.B. $CH_2Cl_2$, $CHCl_3$, Toluol etc. hergestellt. Zur Darstellung der Zielverbindungen 4 werden die geschützten Zwischenprodukte 3 wie allgemein bekannt entblockiert.
So können die $\omega$-Carbonsäurefucoside durch Verseifung der Acylgruppen und der Carbonsäureestergruppen und anschließende Freisetzung der Carbonsäuren aus ihren Salzen hergestellt werden. Die $\omega$-Aminofucoside werden durch Freisetzung der $\omega$-Aminoverbindung aus den blockierten Derivaten und anschließende Verseifung der Acylschutzgruppen bzw. mit umgekehrter Abfolge hergestellt. Die reinen Zielverbindungen 4 werden vorteilhaft durch Kristallisation oder chromatographische Methoden gewonnen.

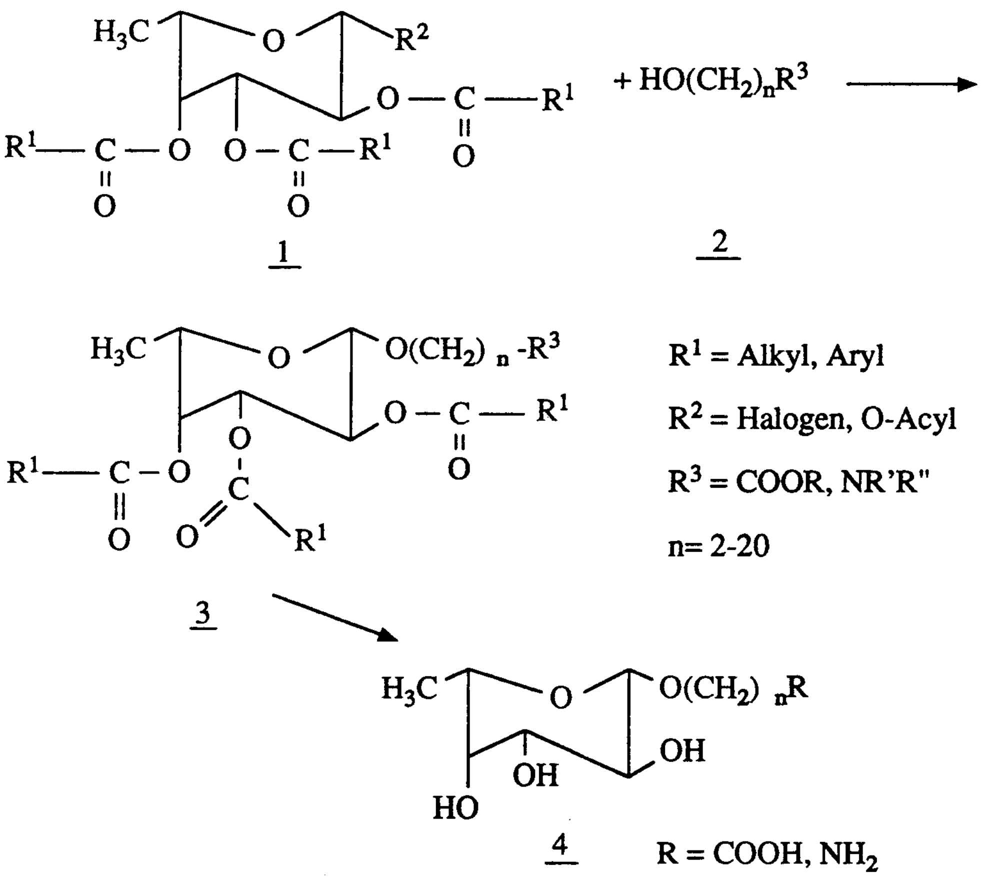

1

$+ HO(CH_2)_nR^3$

2

3

$R^1$ = Alkyl, Aryl

$R^2$ = Halogen, O-Acyl

$R^3$ = COOR, NR'R"

n= 2-20

4

R = COOH, $NH_2$

Alternativ zu 4 wird in Batracylin-konjugaten das $\beta$-L-Fucose-Derivat 5 eingesetzt, das aus dem käuflichen $\beta$-L-Fucose-p-nitrophenylglycosid hydrogenolytisch gewonnen wird.

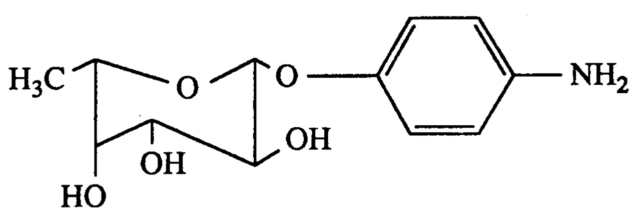

5

Die Synthese von Peptidkongujaten des Batracylins kann nach verschiedenen, in der Peptidchemie bekannten Methoden, erfolgen, die in einem Übersichtswerk beschrieben sind (E. Wünsch in Houben-Weyl, Band 15 1/2 (1974)).

Aus Gründen der Racemisierungsgefahr wird die Einzelschritt-Verlängerung mit urethangeschützten Aminosäuren der Fragmentkondensation vorgezogen.

Die Wahl der Schutzgruppen und der Aktivierungsmethoden läßt vielfältige Variationen zu. Bevorzugt werden bei der Kondensation die Wünsch-Weygand-Methodemit DCC/HOSu oder bei wenig reaktiven Aminofunktionen das Säurechlorid- oder Mischanhydridverfahren eingesetzt.

Die Anknüpfung der L-Fucose-Derivate 4 und 5 kann nach den beschriebenen Methoden an geeignet geschützte Peptidkonjugate des Batracylins erfolgen.

Alternativ dazu können auch zuerst Aminosäure-, Dicarbonsäure- oder Peptidkonjugate von 4 und 5 hergestellt werden, die im weiteren dann mit Batracylin oder Batracylin-Aminosäure-Konjugaten verknüpft werden.

## Beispiele:

### Dipeptidkonjugate:

### Beispiel 1

### H-D-Alanyl-L-Alanyl-Batracylin

Die Synthese läuft ausgehend von Batracylin und Fmoc-Alanin über 4 Reaktionsstufen und wird hier exemplarisch für die Beispiele 1-11 beschrieben.

### Fluorenyl-9-methoxycarbonyl-(Fmoc)-L-alanyl-batracylin

2,5 g (8 mmol) Fmoc-L-Alanin werden in 80 ml abs. Methylenchlorid suspendiert und mit 870 µl (12 mmol) Thionylchlorid versetzt. Nach 30 min wird die gleiche Menge Säurechlorid nachgesetzt und der Ansatz eine weitere Stunde bei Raumtemperatur gerührt. Nach Einengen und Nachdestillieren mit $CH_2Cl_2$ erhält man einen amorphen Feststoff, der sofort weiter umgesetzt wird. Das Fmoc-Aminosäurechlorid wird in 80 ml $CH_2Cl_2$ gelöst und unter Stickstoffatmosphäre in eine Suspension aus 1 g (4 mmol) Batracylin und 500 µl (6 mmol) Pyridin in 400 ml abs. $CH_2Cl_2$ gegeben. Nach 16h Rühren bei Raumtemperatur wird eingeengt und der verbleibende Rückstand 30 min mit Methanol im Ultraschallbad behandelt. Nach Absaugen und intensivem Nachwaschen mit Methanol erhält man 2,07 g (95 %) der Zielverbindung, deren Struktur NMR-spektroskopisch belegt wurde. $R_f$: 0,73 (Toluol/Ethanol 3:1)

### L-Alanyl-batracylin

2 g (3,7 mmol) Fmoc-Alanyl-Batracylin werden in 50 ml Piperidin suspendiert und 30 min im Ultraschallbad behandelt. Man engt ein, destilliert mehrmals mit $CH_2Cl_2$ nach und digeriert den Rückstand mit Methanol. Nach 10 min saugt man ab und wäscht nach, bis der Rückstand piperidinfrei ist. Das Produkt fällt in sehr reiner Form und in nahezu quantitativer Ausbeute an.
Ausbeute: 1,15 g (97%). $R_f$: 0,38 ($CH_3CN/H_2O$/AcOH) 5:1:0,2.

### t-Butyloxycarbonyl-D-alanyl-L-alanyl-batracylin

340 mg (1,8 mmol) Boc-D-Alanin und 340 mg (2,7 mmol) N-Hydroxysuccinimid werden in 30 ml abs. DMF gelöst und bei 0 °C mit 415 mg (2,16 mmol) Dicyclohexylcarbodiimid versetzt. Nach 2h Reaktionszeit bei Raumtemperatur gibt man 513 mg (1,5 mmol) Alanyl-batracylin hinzu und läßt weitere 16h bei Raumtemperatur reagieren. Die Lösung wird aufkonzentriert und der ausgefallene Harnstoff abgetrennt. Das Rohprodukt wird durch Flash-Chromatographie (LM $CH_2Cl_2$/MeOH 99:1) gereinigt. Die entsprechenden Fraktionen werden eingeengt und der Rückstand zur Entfernung vom restlichen Hydroxysuccinimid mit Essigester digeriert. Nach Einengen und Trocknen erhält man das geschützte Dipeptidkonjugat, dessen Struktur NMR-spektroskopisch belegt wird.
Ausbeute: 570 mg (72 %) $R_f$: 0,65 (Toluol/Ethanol 3:1)

### D-Alanyl-L-alanyl-batracylin

570 mg (1,16 mmol) des geschützten Dipeptidkonjugats Boc-D-Ala-L-Ala-Batracylin werden bei 0 ° C mit 4,5 ml wasserfreier Trifluoressigsäure behandelt. Einengen, Nachdestillieren mit Wasser und Geftiertrocknen ergibt in quantitativer Ausbeute das Trifluoracetat der Zielverbindung.
$R_f$: 0,35 ($CH_3CN/H_2O$/AcOH 5:1:0,2).

Folgende Verbindungen werden in Analogie zu 1 hergestellt:

Beispiel 2

H-L-Ala-L-Ala-Batracylin

$R_f$: 0,35 ($CH_3CN/H_2O$/AcOH 5:1:0,2)

Beispiel 3

H-D-Ala-D-Ala-Batracylin

$R_f$: 0,35 ($CH_3CN/H_2O$/AcOH 5:1:0,2)

Beispiel 4

H-D-Glu(Bzl)-L-Ala-Batracylin

$R_f$: 0,7 ($CH_3CN/H_2O$/AcOH 5:1:0,2)
Terminaler Baustein: Boc-D-Glu(Bzl)OH

Beispiel 5

H-Lys-Ala-Batracylin

$R_f$: 0,42 ($CH_3CN/H_2O$/AcOH 10:5:3)
Terminaler Baustein: Boc-Lys(Boc)OH

Beispiel 6

H-Tyr-Ala-Batracylin

$R_f$: 0,47 ($CH_3CN/H_2O$/AcOH 5:1:0,2)
Terminaler Baustein: Boc-Tyr-OH

Beispiel 7

Z-Lys-Ala-Batracylin

$R_f$: 0,4 ($CH_3CN/H_2O$/AcOH 5:1:0,2)
Terminaler Baustein: Z-Lys(Boc)-OH

Beispiel 8

H-Lys(Boc)-Ala-Batracylin

$R_f$: 0,46 ($CH_3CN/H_2O$/AcOH) 5:1:0,2)
Terminaler Baustein: Fmoc-Lys(Boc)-OH. Die Synthese erfolgt in Analogie zu Beispiel 1 mit Ausnahme der 4.Stufe: Die Deblockierung der terminalen Aminofunktion wird hier durch eine zweite Fmoc-Abspaltung mit Piperidin, wie sie in Beispiel 1 für die Darstellung von Alanyl-Batracylin beschrieben wurde, erreicht.

Beispiel 9

Boc-Lys-Ala-Batracylin

$R_f$: 0,42 ($CH_3CN/H_2O$/AcOH 5:1:0,2)
Terminaler Baustein: Boc-Lys(Fmoc)OH
(Synthese wie Beispiel 8)

Beispiel 10

Fmoc-Glu-Ala-Batracylin

Terminaler Baustein: Fmoc-Glu(tBu)-Batracylin

Beispiel 11

H-Glu-Ala-Batracylin

$R_f$: 0,24 ($CH_3CN/H_2O$/AcOH 5:1:0,2)
Fmoc-Abspaltung aus 10

Glycopeptidkonjugate

Beispiel 12

β-L-Fuc-Hyac-D-Ala-L-Ala-Batrayclin

β-L-Fuc(Ac)$_3$Hyac-OMe

Ein Gemisch aus 2,2 g (24,3 mmol) Hydroxyessigsäuremethylester 1,3 g pulverisiertem Molekularsieb 4Å und 3,8 g Quecksilber(II)-cyanid in 50 ml Dichlormethan wird auf -20°C abgekühlt. Eine Lösung des peracetylierten 2,3,4-Tri-O-acetyl-α-L-fucopyranosylbromid (7,0 g) in 50 ml Dichlormethan wird zugetropft und anschließend bei Raumtemperatur gerührt. Die Umsetzung wird durch Dünnschichtchromatographie (Laufmittel: Toluol-Essigsäureethylester 1:1 ν/ν $R_f$ (1) = 0,75, $R_f$ (2) = 0,61) verfolgt. Nach Beendigung der Reaktion wird mit Dichlormethan verdünnt, abfiltriert und das Filtrat mit gesättigter Kaliumiodid-Lösung und Wasser gewaschen. Die organische Phase wird dann über Magnesiumsulfat getrocknet und eingedampft. Das Rohprodukt wird schließlich über 700 g Kieselgel (Merck 7734) mit Toluol-Essigsäureethylester 5:1 (ν/ν) als Laufmittel gereinigt. Man erhält 3 g Produkt (55 % der Theorie) als sirupöse Masse.

β-Fuc-Hyac-OH

β-Fuc(Ac)$_3$Hyac-OMe wird in 34 ml wasserfreiem Methanol gelöst und mit 1 ml 1n Natriummethanolat-Lösung in Methanol versetzt. Nach 4 Stunden bei Raumtemperatur wird mit 30,5 ml einer wäßrigen 1N Natronlauge versetzt und die Verseifung dünnschichtchromatographisch verfolgt. (Laufmittel: Toluol-Ethanol 3:1, ν/ν $R_f$ (2) = 0,98, $R_f$ (3) = 0,34). Nach Beendigung der Reaktion wird mit Ionenaustauscher SC 108 ($H^+$) neutralisiert, filtriert und das Filtrat eingedampft. Das Rohprodukt wird schließlich über eine Ionenaustauschersäule mit 150 g Ionenaustauscher SC 108 ($H^+$) mit Wasser eluiert. Die produktenthaltenden Fraktionen werden eingedampft und das reine Produkt gewonnen. Ausbeute: 1,4 g (75 % der Theorie).

β-L-Fuc-Hyac-D-Ala-L-Ala-Batrayclin

200 mg (0,9 mmol) β-L-Fuc-Hyac werden in 30 ml DMF aufgenommen und mit 208 mg (1,8 mmol) N-Hydroxysuccinimid versetzt. Man gibt bei 0°C 223 mg (1,1 mmol) DCC zu und läßt langsam auf Raumtemperatur erwärmen. Nach 3h werden 455 mg (0,9 mmol) des Dipeptidkonjugats aus Beispiel 1 zugegeben. Nach 16h Reaktionszeit wird der ausgefallene Harnstoff abgetrennt und das Filtrat eingeengt. Nach Digerieren mit Methanol und Umfällen aus DMF/MeOH/Isoprop. erhält man 250mg (47 %) der sauberen Zielverbindung:
$R_f$: 0,7 ($CH_3CN/H_2O$/AcOH 5:1:0,2).

Die Verbindungen 13-25 werden analog nach dieser Vorschrift hergestellt. In den Beispielen 20-25 wird an Stelle der Hydroxyessigsäure ω-Hydroxycaprylsäure eingesetzt.

Beispiel 13

β-L-Fuc-Hyac-Lys(Boc)-Ala-Batracylin

$R_f$: 0,43 ($CH_3CN/H_2O$ 10:1)
Kupplungsbaustein: H-Lys(Boc)-Ala-Batracylin

Beispiel 14

β-L-Fuc-Hyac-Lys-Ala-Batracylin

$R_f$: 0,2 ($CH_3CN/H_2O/AcOH$ 5:1:0,2)
(Deblockierung der Verbindung aus Beispiel 13)

Beispiel 15

H-Lys(β-L-Fuc-Hyac)-Ala-Batracylin

$R_f$: 0,18 ($CH_3CN/H_2O/AcOH$ 5:1:0,2)
(Kupplungsbaustein: Boc-Lys(H)-Ala-Batracylin

Beispiel 16

β-L-Fuc-Hyac-Lys(β-L-Fuc-Hyac)-Ala-Batracylin

$R_f$: 0,5 ($CH_3CN/H_2O$ 5:1:0,2)

Beispiel 17

Z-Lys(β-L-Fuc-Hyac)-Ala-Batracylin

$R_f$: 0,5 ($CH_3CN/H_2O$ 10:1)
(Kupplungsbaustein: Z-Lys(H)-Ala-Batracylin)

Beispiel 18

β-L-Fuc-Hyac-Glu-Ala-Batracylin

$R_f$: 0,1 ($CH_3CN/H_2O$ 10:1)
(Kupplungsbaustein: H-Glu(tBu)-Ala-Batracylin;
t-Butylester-Abspaltung analog zur Boc-Abspaltung in Beispiel 1)

Beispiel 19

β-L-Fuc-Hyac-Tyr(OH)-Ala-Batracylin

$R_f$: 0,78 ($CH_3CN/H_2O/AcOH$ 5:1:0,2)

Beispiel 20

Boc-Lys(β-L-Fuc-Hycapr)-Ala-Batracylin

$R_f$: 0,48 ($CH_3CN/H_2O$ 10:1)

Beispiel 21

H-Lys(β-L-Fuc-Hycapr)-Ala-Batracylin

$R_f$: 0,45 ($CH_3CN/H_2O/AcOH$ 10:3:1,5)
(Kupplungsbaustein: Boc-Lys-Ala-Batracylin)

Beispiel 22

β-L-Fuc-Hycapr-Lys-Ala-Batracylin

(Kupplungsbaustein: H-Lys(Boc)-Ala-Batracylin)

Beispiel 23

β-L-Fuc-Hycapr-Lys(β-L-Fuc-Hycapr)-Ala-Batracylin

(Kupplungsbaustein: H-Lys-Ala-Batracylin)

Beispiel 24

β-L-Fuc-Hycapr-D-Ala-L-Ala-Batracylin

Beispiel 25

β-L-Fuc-Hycapr-Glu-Ala-Batracylin

(Kupplungsbaustein: H-Glu(tBu)-Ala-Batracylin)

Beispiel 26

β-L-Fuc-pAP-Suc-Ala-Batracylin

β-L-Fuc-pAP:

170 mg (0,67 mmol) β-(p-Aminophenyl)-L-fucosid werden in 10 ml DMF mit 67 mg (0,67 mmol) Bernsteinsäureanhydrid versetz. Nach 2h Rühren bei RT engt man ein und fällt aus Isopropanol/Ether das Produkt aus.
Ausbeute: 200 mg (85 %) $R_f$: 0,15 ($CH_3CN:H_2O$ 10:1)
Die Verknüpfung mit Alanyl-Batracylin wird wie in Beispiel 1 beschrieben in Gegenwart von DCC/HOSu durchgeführt.
Ausbeute: 265 mg (72 %) $R_f$: 0,44 ($CH_3CN/H_2O$ 10:1).

Die Beispiele 27 und 28 werden analog zu dieser Vorschrift hergestellt.

Beispiel 27

β-L-Fuc-pAP-Suc-Val-Batracylin

Beispiel 28

β-L-Fuc-pAP-Glut-Ala-Batracylin

Beispiel 29

β-L-Fuc-ω-AHex-Suc-Ala-Batracylin

β-L-Fuc($Ac_3$)ω-(N-Z-AHex)

Glycosylierungsreaktion in Analogie zu Beispiel 12, ausgehend von peracetyliertem L-Fucosylbromid und N-Benzyloxycarbonyl-ω-aminohexanol, das in bekannter Weise hergestellt werden kann.

β-L-Fuc-ω-AHex

Deblockierung wie in Beispiel 12, gefolgt von hydrogenolytischer Z-Gruppen-Abspaltung.

β-L-Fuc-ω-AHex-Suc-OH

Umsetzung mit Bernsteinsäureanhydrid wie in Beispiel 26

β-L-Fuc-ω-AHex-Suc-Ala-Batracylin

Kupplungsreaktion wie in Beispiel 1 beschrieben
Die Beispiele 30 und 31 werden analog hergestellt.

Beispiel 30

H-Glu(β-Fuc-ω-AHex)-Ala-Batracylin

(Kupplungsbaustein: Boc-Glu-Ala-Batracylin)

Beispiel 31

H-Glu(β-Fuc-pAP)-Leu-Batracylin

(Kupplungsbaustein: Boc-Glu-Leu-Batracylin)

Material und Methoden zur biologischen Testung

Die Zellinie SW 480 (CCL 228) stammt aus einem humanen Adenocarcinom des Dickdarms. Sie wurde in der Zeit von 1971-1975 von A. Leibovitz isoliert und wird von ATCC in der 96. Passage geliefert.

In den Experimenten wurde sie in der 104. bis zur 111. Passage eingesetzt. Als Nährmedium diente L 15 (Gibco) mit 10 % foetalem Kälberserum (L 15/FCS 10). Die Kontrollzellinie BHK wurde in DMEM/FCS10 Medium angezogen. Sie befand sich in der Passage 109-116.

Kulturbedingungen für Wachstumsinhibitionteste

SW 480 Zellen wurden in Rouxschalen in L15/FCS10 Medium gezogen. 24 Stunden vor Gebrauch wurde das Medium gewechselt und zusätzlich 0,1 % BSA zugesetzt. Anschließend wurde typsiniert und in MEME (Flow) unter Zusatz von 10 % FCS und 0,1 % BSA zu einer Zellzahl von 100 000 Zellen/ml aufgenommen. 100 µl Zellsuspension/Well wurden in eine 96 Mikrowellplatte gegeben und 3 Tage bei 37° C im $CO_2$ Brutschrank inkubiert. Anschließend wurde 100 µl MEME Medium in 1 µl DMSO mit den Prüfsubstanzen (Glycokonjugaten) zugesetzt. Das Wachstum wurde täglich überprüft.

Konfluente Vorkulturen von BHK wurden trypsiniert und in GMEM (Flow) Medium im 10 % Tryptose (Gibco), 10% Newborn Calf Serum, 0,5 % L-Glutamin-Lösung 100 x (Gibco), 0,75 % Penicillin-Streptomycin-Lösung (Gibco) und 0,1 % BSA zu einer Zellzahl von 100 000 Zellen/ml aufgenommen. 100 µl Zellsuspension/Well wurden in 96 Well/Mikrotiterplatten gegeben und 24h bei 37° C im $CO_2$ Brutschrank inkubiert.

Anschließend wurden 100 µl GMEM-Medium und die Prüfsubstanzen in 1 µl DMSO pro Mikrowell zugesetzt. Das Wachstum wurde täglich überprüft.

Wachstumsinhibitionsteste

Zu jedem Mikrowell wurde alle 24h 40 µl MTT-Lösung (3-[4,5-Dimethylthiazol-2-yl]-2,5-diphenyltetrazoliumbromid) mit einer Ausgangskonzentration von 5 mg/ml $H_2O$ zugesetzt. Es wurde 5 Stunden im $CO_2$ Brutschrank bei 37° C inkubiert. Anschließend wurde das Medium abgesaugt und 100 µl i-Propanol/Well zugesetzt. Nach 30 min Schütteln wurde die Extinktion bei 545 nm mit einem Titertek Multiskan MCC/340 (Flow) gemessen.

**Patentansprüche**

**1.** Verbindungen der allgemeinen Formel

$[R]_n—AA_1—AA_2—C$

mit

C = ein Cytostatikum,

R =

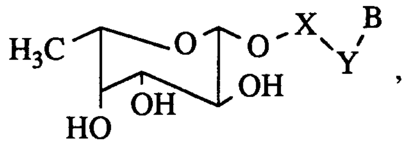

X = Alkylen, Arylen

Y = CO,NH,O

B = Bindung oder Diacylrest einer Dicarbonsäure,

$AA_1$ = ein natürlicher Aminosäure-Rest in der L- oder D-Konfiguration oder Bindung

und

$AA_2$ = ist eine natürliche Aminosäure in der D- oder L-Konfiguration oder Bindung,

n = 0,1,2.

**2.** Verbindungen gemäß Anspruch 1

worin bedeutet

X = p-Phenylen oder$(CH_2)_m$,
wobei m 1-20 sein kann

Y = CO,NH,

B = Bindung oder Diacylrest einer $C_3$-$C_6$-Dicarbonsäure,

n = 0,1,2.

**3.** Verbindungen gemaß den Ansprüchen 1 und 2

worin bedeutet

$AA_1$ = Aminosäure-Reste mit sauren oder basischen Seitenketten oder Bindung,

$AA_2$ = Aminosäure-Reste wie Ala, Val, Leu, Ile, Nva, Gly, Phe (D oder L-Konfiguration), die amidartig mit dem Heterocyclus und mit $AA_1$ verknüpft sind.

**4.** Verbindungen gemäß den Ansprüchen 1 bis 3 worin C für

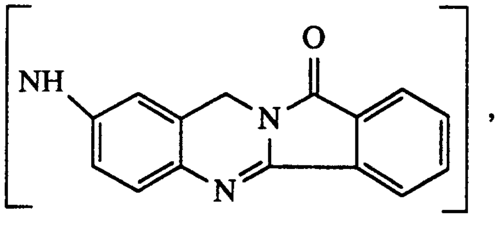

steht.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 10 2449

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| A | EP-A-0 365 956 (BAYER)<br>* Das ganze Dokument * & US-A-4 980 343 (BAYER) (Kat. D)<br>--- | 1-4 | C 07 K 9/00<br>C 07 D 487/04<br>A 61 K 37/02 |
| A | EP-A-0 359 347 (NEORX)<br>* Ansprüche *<br>--- | 1-4 | |
| A | THE JOURNAL OF ANTIBIOTICS, Band XLII, Nr. 12, Dezember 1989, Seiten 1823-1830, Toyko, JP; J.C. FLORENT et al.: "Synthesis and antitumor activity of new anthracyclines"<br>* Das ganze Dokument *<br>----- | 1-4 | |

RECHERCHIERTE SACHGEBIETE (Int. Cl.5)

C 07 K
C 07 D
A 61 K

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**

**Vollständig recherchierte Patentansprüche:** 4
**Unvollständig recherchierte Patentansprüche:** 1-3
**Nicht recherchierte Patentansprüche:**
**Grund für die Beschränkung der Recherche:**

Das Glied C der Formel im Anspruch I ist unklar definiert, deswegen wurde die Recherche nur auf C = Batracylin beschränkt.

| Recherchenort | Abschlußdatum der Recherche | Prüfer |
|---|---|---|
| DEN HAAG | 28-05-1992 | MASTURZO P. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0409)